# EUROPEAN PATENT SPECIFICATION

(11) **EP 1 024 824 B1**
(45) Date of publication and mention of the grant of the patent: **01.09.2004**
(21) Application number: 98950200.0
(22) Date of filing: 27.10.1998
(51) Int. Cl.: A61K 39/00, A61K 9/00

(54) **SOLID DISPERSING VACCINE COMPOSITION FOR ORAL DELIVERY**
FESTE DISPERGIERENDE IMPFSTOFFE-ZUSAMMENSETZUNG ZUR ORALEN VERABREICHUNG
COMPOSITION DE VACCIN SOLIDE DISPERSANTE S'ADMINISTRANT PAR VOIE ORALE

(30) Priority: 27.10.1997 GB 9722682
(43) Date of publication of application: 09.08.2000
(73) Proprietor: R.P. SCHERER CORPORATION, Troy, Michigan 48007-7060 (US)
(72) Inventor: SEAGER, Harry, Corsham, Wiltshire SN14 9AN (GB)
(74) Representative: Pearce, Anthony Richmond
(86) International application number: PCT/GB1998/003209
(87) International publication number: WO 1999/021579

(56) References cited:
- WO-A-94/20070
- WO-A-96/21464
- GB-A- 1 548 022
- US-A- 5 298 261
- MESTECKY J ET AL: "BIODEGRADABLE MICROSPHERES FOR THE DELIVERY OF ORAL VACCINES" JOURNAL OF CONTROLLED RELEASE, vol. 28, no. 1/03, 1 January 1994, pages 131-141, XP000435244

## Description

This invention relates to pharmaceutical products, and particularly to such products suitable for oral ingestion and capable of rapid disintegration in the oral cavity.

A large variety of dosage forms for oral ingestion are known and readily available. Such dosage forms are used for the controlled delivery of medicaments to different parts of the body, the requisite control being achieved by the rate at which the carrier for the medicament breaks down and releases it. Thus, fast dispersing carriers are used for such products in which the medicament is to be quickly released. Slower dispersing carriers and carriers resistant to digestion by gastro-intestinal tract glands can be/are used where it is intended that release of the medicament is to be delayed, for example until the product has itself reached the stomach or lower intestine.

Vaccines, which are important in prophylaxis against disease, exert their effects by provoking an immune response, the effect of which is to prevent re-infection by the challenging organism, or the onset of the disease process which would otherwise occur when the antigen against which the immune response has been provoked again challenges a sensitive tissue.

Most existing vaccines are delivered by injection, which is traumatic, inconvenient, expensive, and may fail to induce an appropriate immunogenic response in the mucosal tissues. Delivery by injection does not of course directly target the mucosal surfaces.

W094/20070 discloses an immunisation composition comprising an immunising amount of an antigen and a mucoadhesive in an amount sufficient to induce or enhance immune response to the antigen. Such composition may be administered orally, nasally, rectally or by means of a swab on the tonsil.

The present invention is directed at the use of oral dosage forms of the kind described above to carry vaccines to sites in the human or animal body where they can be best absorbed in a manner which promotes an immune response. According to the invention, there is provided a vaccine composition comprising an immunogenic amount of an antigenic preparation in an orally administered solid dispersing form designed to disintegrate rapidly in the oral cavity, the composition including an adjuvant to enhance absorption of the antigen at the mucosal tissues of the buccopharyngeal, wherein said adjuvant comprises biodegradable polymeric material particles in which the antigenic preparation is encapsulated or on which the antigenic preparation is absorbed, characterised in that in use said solid dispersing form disintegrates rapidly in the oral cavity whereby to coat and be retained in contact with the mucosal tissue of the buccophargyngeal region prior to absorption by said mucosal tissue.

After disintegration, the components of the dosage form rapidly coat and are retained in contact with the mucosal tissues of the buccopharyngeal region, to include mucosal associated lymphoid tissue. This brings the antigenic components in contact with tissues capable of absorption of the antigen. The dosage form may further incorporate an adjuvant which serves to potentiate the immunogenic response upon absorption. It is preferred that compositions of the invention disintegrate within 1 to 60 seconds, more preferably 1 to 30 seconds, especially 1 to 10 seconds and particularly 2 to 8 seconds, of being placed in the oral cavity. Normally, the disintegration time will be less than 60 seconds following the disintegration method specified in United States Pharmacopoeia No. 23, 1995, in water at 37°C. Longer disintegration times may be needed if bioadhesive polymers are used to extend the residence time. However, normally disintegration within one minute should be possible.

80% of infections affect, or start in, the mucosal surfaces. Active immunisation against these infective agents may depend on the successful induction of a mucosal immune response. Successful mucosal vaccines could both protect the secretory surfaces- mucosal immunity, and also induce systemic immunity by induction of circulatory antibodies. They also have the benefit of ease of administration and lower costs. The induction of mucosal immunity is evidenced by the appearance of immunoglobulin A antibodies (IgA) in the mucous overlying the mucosa. Successful local stimulation of the mucosal membrane system produces a barrier against a specific pathogen, but this adaptive immunity also confers protection to mucous membranes at other sites in the body. Potentially, oral vaccines can be used to induce immunity against oral, respiratory, genital and ocular pathogens. This ability to generate immunity at sites in the body away from the point of original antigenic stimulation has led to the concept of a common mucosal immune system. There are further indications that stimulation of the mucosal immune system can induce protective circulatory antibodies in the systemic immune system particularly IgG antibodies.

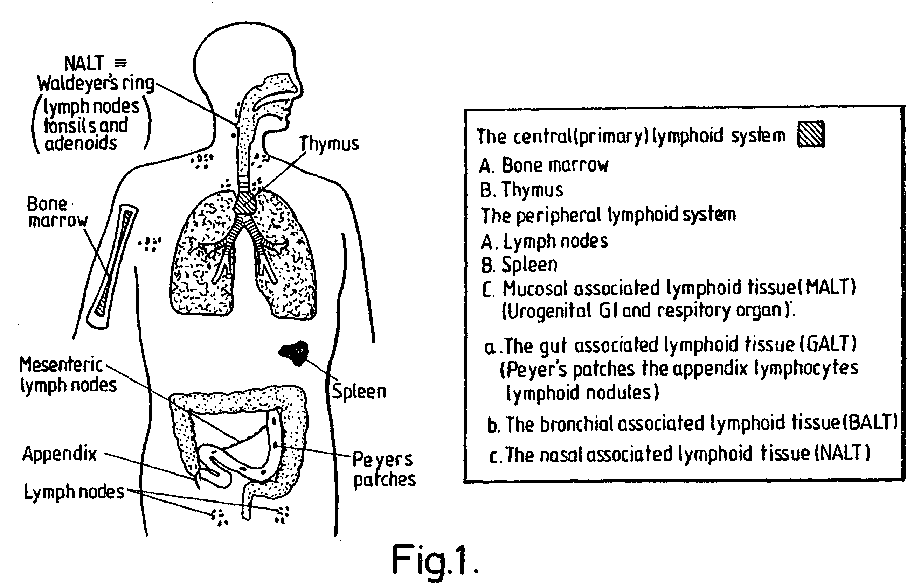

Vaccines delivered orally could stimulate nasal associated lymphoid tissue in the mouth and nasal pharyngeal area - the lymph nodes, tonsils and adenoids, and the gut associated lymphoid tissue in the Peyer's patches in the small intestine. Figure 1 appended hereto illustrates the location of these tissues.

Vaccines incorporate antigens which can be peptides, proteins or whole or partial fragments or extracts of bacterial or viral cells often attenuated to remove toxic components. A primary problem in vaccination procedures is ensuring that these antigens or antigenic compounds reach the appropriate site in sufficient quantities to provoke the requisite immune response.

There are two elements of the immune system which can provide the requisite immune response when stimulated by an antigen in a vaccine system. These are the systemic immune system and the mucosal immune system. The latter consists of areas of lymphoid tissue located in the gastrointestinal tract, the respiratory tract, the genitourinary tract, and the membranes surrounding sensory organs, and it is with this immune system that the present invention is particularly concerned. We have found that these areas of lymphoid tissue are very effectively targeted by antigens carried on a water dispersable matrix placed on the tongue. Such localised areas of lymphoid tissue, when activated by an absorbed antigen, secrete immunogloblin A (IgA), which exerts an important function in mucosal immunity. Secretory IgA molecules resist proteolysis and mediate antibody-dependant T cell mediated cytotoxicity; inherant microbal adherence, colonisation and penetration, as well as food antigen uptake. Stimulation of mucosal tissue can also result in secretion of circulatory IgG antibodies and in turn, IgM and IgE antibodies.

The principal function of the cells forming the lymphoidal tissue is to prevent absorption of pathogens and toxins or to inactivate these pathogens and toxins upon absorption to mucosal tissue. Generally considerably higher doses of antigens are required for mucosal immunisation, especially by the oral route. This is due to the existence of effective mechanical and chemical barriers, and the degradation and digestion of antigens by enzymes and acids. Additionally, there is a rapid clearance of material from the upper respiratory and digestive tracts to the stomach by mucociliary, peristatic and secretory processes.

Rapidly dispersing oral administered dosage forms can be taken without water and disperse in very small volumes of saliva. This increases the coating of mucosal tissues containing the tonsillar associated lymphoid tissue and increases the residence time of antigens with these tissues. Some fast dispersing dosage forms are inherently mucoadhesive but residence time in contact with the target tissue could be further enhanced by the incorporation of a specific mucoadhesive. Suitable mucoadhesives are detailed in European patent application 92109080.9 (EP-A-0 516 141) and include:
. polyacrylic polymers such as carbomer and carbomer derivatives (Polycarbophyl, Carbopol etc);
. cellulose derivatives such as hydroxypropylmethylcellulose (HPMC), hydroxythylcellulose (HEC), hydroxypropylcellulose (HPC) and sodium carboxymethylcellulose (NaCPC);
. natural polymers such as gelatin, sodium alginate, pectin;

Suitable commercial sources for representative bioadhesive polymers include:
Carbopol acrylic copolymer - BF Goodrich
Chemical Co., Cleveland, OH, USA
HPMC - Dow Chemical Col, Midland, MI, USA
HEC (Natrosol) - Hercules Inc., Wilmington, DE, USA
HPC (Kluoel) - Dow Chemical Co., Midland, MI, USA
MaCMC - Hercules, Inc., Wilminton, DE, USA
Gelatin - Deamo Chemical Corp., Elmford, NY, USA
Sodium Alginate - Edward Mandell Co., Inc., Carmel, NY, USA
Pectin - BDH Chemicals Ltd., Poole, Dorset, UK
Polycarbophil - BF Goodrich Chemical Co.,
Cleveland, OH, USA

The rapid dissolving dosage form promotes delivery of the vaccine to the target site, and the mucoadhesive system can be designed to maintain the vaccine in contact with the target mucosal lymphoid tissues in the mouth and pharynx, and to increase the residence time of the vaccine element at these potential surfaces for absorbtion. As a product for oral ingestion, from which the vaccine is quickly released once the product is taken, high concentrations of vaccine can thus be quickly delivered to the desired target sites.

Further adjuvants may be used to enhance absorption of the antigen at the target lymphoid tissue and/or to potentiate the immune response resulting from this absorption and stimulation. Suitable adjuvants can include the following:
Aluminium salts, non-toxic bacterial fragments, cytokines, cholera toxin (and detoxified fractions thereof), Chitosan, Homologous heat-labile of E.coli (and detoxified fractions thereof), Lactide/glycolide homo ± and copolymers (PLA/GA), Polyanhydride e.g. trimellitylimido-L-tyrosine, DEAE-Dextran, Saponins complexed to membrane protein antigens (immune stimulating complexes - ISCOMS), Bacterial products such as lipopolysaccharide (LPS) and Muramyl dipeptide, (MDP), Liposomes, Cochleates, Proteinoids, Cytokines (Interleukins, interferons), Genetically engineered live microbial vectors, Non-infectious pertussis mutant toxin, Neurimidase/Galactose oxidase, Attenuated bacterial and viral toxins derived from mutant strains.

In some preferred embodiments of the invention, the biodegradable polymeric material particles may be microspheres onto or into which the vaccine element is absorbed or incorporated, rendering it available for absorption into the lymphoid tissue effectively as soon as the tissue is ready to receive it. Particularly suitable biodegradable polymeric materials are hydrophobic materials such as poly(lactic acid) and poly(lactide-co-glycide) polymers, and latex copolymers. These polymeric materials also confer resistance to enzymatic and hydrolytic digestion until their absorption into lymphoid tissue where the antigen can exert its immogenic effect. They are preferentially made of hydrophobic materials which enhance absorption into the target tissues.

Fast dispersing dosage forms, delivered orally, have been demonstrated to rapidly disperse and coat the mucosal surfaces in the mouth and pharynx, where the mucosal associated lymphoid tissues are localised. In this respect reference is directed to a paper by Wilson et al published in the International Journal of Pharmaceutics, 40 (1997) pages 119-123. Figure 1 in that paper shows the results of a gamma scintigraphic study. Dosage forms which dissolve rapidly in saliva, without the aid of water, have also been demonstrated to increase the time in which the rapidly dispersed contents are in contact with the target lymphoid tissue within the buccopharyngeal area and increase the time . taken to reach the stomach, when compared to tablets and capsules. Reference is directed to a further paper by wilson et al published in the International Journal of Pharmaceutics, 46 (1998) pages 241-246); see particularly Figure 1.

The use of a fast-dispersing dosage form, delivered orally, improves the targeting of vaccines to susceptible lymphoid tissues in the mouth and the pharynx, and in turn, the concentration of vaccine making contact with these tissues. Fast-dispersing dosage forms increase the contact time of vaccines with the susceptible lymphoid tissue in the buccopharyngeal area.

One example of a fast-dispersing dosage form is described in U.S. Patent No. 4855326 in which a melt spinnable carrier agent, such as sugar, is combined with an active ingredient and the resulting mixture spun into a "candy-floss" preparation. The spun "candy-floss" product is then compressed into a rapidly dispersing, highly porous solid dosage form.

U.S. Patent No. 5120549 discloses a fast-dispersing matrix system which is prepared by first solidifying a matrix-forming system dispersed in a first solvent and subsequently contacting the solidified matrix with a second solvent that is substantially miscible with the first solvent at a temperature lower than the solidification point of the first solvent, the matrix-forming elements and active ingredient being substantially insoluble in the second solvent, whereby the first solvent is substantially removed resulting in a fast-dispersing matrix.

U.S. Patent No. 5079018 discloses a fast-dispersing dosage form which comprises a porous skeletal structure of a water soluble, hydratable gel or foam forming material that has been hydrated with water, rigidified in the hydrated state with a rigidifying agent and dehydrated with a liquid organic solvent at a temperature of about 0°C or below to leave spaces in place of hydration liquid.

Published International Application No. WO 93/12769 (PCT/JP93/01631) describes fast-dispersing dosage forms of very low density formed by gelling, with agar, aqueous systems containing the matrix-forming elements and active ingredient, and then removing water by forced air or vacuum drying.

U.S. Patent No. 5298261 discloses fast-dispersing dosage forms which comprise a partially collapsed matrix network that has been vacuum-dried above the collapse temperature of the matrix. However, the matrix is preferably at least partially dried below the equilibrium freezing point of the matrix.

Published International Application No. WO 91/04757 (PCT/US90/05206) discloses fast-dispersing dosage forms which contain an effervescent disintegration agent designed to effervesce on contact with saliva to provide rapid disintegration of the dosage form and dispersion of the active ingredient in the oral cavity.

U.S. Patent No. 5595761 discloses a particulate support matrix for use in making a rapidly dissolving tablet, comprising;
a first polypeptide component having a net charge when in solution, e.g. non-hydrolysed gelatin;
a second polypeptide component having a net charge of the same sign as the net charge of the first polypeptide component when in solution e.g. hydrolysed gelating; and
a bulking agent, and wherein the first polypeptide component and the second polypeptide component together comprise about 2% to 20% by weight of the particulate support matrix and wherein the bulking agent comprises about 60% to 96% by weight of the particulate support matrix; and
wherein the second polypeptide component has a solubility in aqueous solution greater than that of the first polypeptide component and wherein the mass: mass ratio of the first polypeptide component to the second polypeptide component is from about 1 : ½ to about 1 : 14; and
wherein when the support matrix is introduced into an aqueous environment the support matrix is disintegrable within less than about 20 seconds.

US Patent No. 5576014 discloses fast-dispersing dosage forms which dissolve intrabuccally and which comprise compressed moldings formed from granules comprising a saccharide having low moldability which has been granulated with a saccharide having high moldability. The resulting compressed moldings show quick disintegration in the buccal cavity.

EP-B-0690747 describes particles comprising an excipient forming a matrix and at least one active ingredient uniformly distributed in the mass of the matrix which are prepared by a process comprising the steps of preparing an homogeneous pasty mixture with a viscosity below 1 Pa.s, measured at room temperature (15-20°C), from at least one active ingredient, a physiologically acceptable hydrophilic excipient and water; extruding the resulting homogenous mixture and cutting the extrudate to give moist particles; freezing the resulting particles as they fall under gravity through a stream of inert-gas at a temperature below 0°; and drying the particles by freeze drying.

Australian Patent No. 666666 discloses a rapidly disintegratable multiparticulate tablet having a mixture of excipients in which the active substance is present in the form of coated microcrystals or optionally coated microgranules. Such tablets disintegrate in the mouth in an extremely short time, typically less than 60 seconds.

US Patent No. 5382437 discloses a porous carrier material having sufficient rigidity for carrying and administering an active material which is capable of rapid dissolution by saliva and which is formed by freezing a liquified ammonia solution comprising liquid ammonia, a liquid ammonia-soluble gel or foam material and a rigidifying agent for the gel or foam material selected from the group consisting of a monosacchanide, a polysacchanide and combinations thereof, and deammoniating the frozen material thus formed by causing material transfer of ammonia from the frozen state to the gas state thereby leaving spaces in the carrier material in place of the frozen ammonia.

Published International Application No. WO 93/13758 (PCT/US92/07497) describes tablets of increased physical strength which disintegrate in the mouth in less than 10 seconds and which are prepared by combining and compressing a meltable binder, excipients and a pharmaceutically active agent into a tablet, melting the binder in the tablet and then solidifying the binder.

US Patents Nos. 3885026 and 4134943 also disclose fast-dispersing porous tablets and a method for increasing their physical strength by first compressing the tablet and then volatilising a readily volatilisable solid adjuvant incorporated in the tablet to attain the desired porosity.

EP-A-0601965 describes a shearform matrix material which can be used, inter alia, to deliver a pharmaceutically active agent. The shearform matrix is formed by increasing the temperature of a feedstock which includes a solid non-solubilised carrier material to the point where it will undergo internal flow with the application of a fluid shear force, ejecting a stream of the heated feedstock thus formed under pressure from an orifice and then subjecting the feedstock to disruptive fluid shear force which separates the flow of feedstock into multiple parts and transforms the morphology of the feedstock.

US Patent No. 5683720 discloses discrete particles containing a pharmaceutically active agent which can be fast-dispersing and are formed by subjecting a solid, organic feedstock to liquiflash conditions whereby the feedstock is transformed instantaneously from solid to liquiform to solid, liquiform being a transient condition in which the feedstock has substantially unimpeded internal flow. Shear force is then imparted to the liquiform feedstock in an amount sufficient to separate tiny masses of feedstock which then solidify as discrete particles.

US Patent No. 5576014 discloses fast-dispersing dosage forms in the form of intrabuccally dissolving compressed mouldings comprising a saccharide having low mouldability which has been granulated with a saccharide having high mouldability.

Published International Application No. WO 95/34293 describes the preparation of fast-dispersing dosage forms comprising a three-dimensional crystalline-based porous network bound together to form a stable structure which is formed by mixing uncured shearform matrix and an additive, moulding the dosage form and curing the shearform matrix.

EP-A-0737473 discloses fast-dispersing dosage forms which are effervescent. Each such dosage form comprises a mixture of at least one water or saliva activated effervescent agent and a plurality of microcapsules containing the active ingredient.

US Patent No. 5587180 describes fast-dispersing dosage forms which include an active ingredient and a particulate support matrix comprising a first polymeric component which may be a polypeptide such as a non-hydrolysed gelatin, a second polymeric component which may be a different polypeptide such as a hydrolysed gelatin and a bulking agent. Generally, the dosage forms are prepared by mixing the particulate support matrix with the active ingredient and any other additives and then forming the mixture into tablets by compression.

EP-A-0627218 discloses a fast-dispersing dosage form which comprises a tablet comprising a sugar alcohol or the like as principal ingredient which is prepared by the wet granulation method in which a kneaded mixture of the sugar alcohol or the like with a drug is compression molded before drying.

Published International Application No. WO 94/14422 describes a process for drying frozen discrete units in which the solvent is removed under conditions whereby the solvent is evaporated from the solid through the liquid phase to a gas, rather than subliming from a solid to a gas as in lyophilisation. This is achieved by vacuum drying at a temperature below the equilibrium freezing point of the composition at which point the solvent (such as water) changes phase.

The term "fast-dispersing dosage form" refers to compositions which disintegrate/disperse within 1 to 60 seconds, preferably 1 to 30 seconds, more preferably 1 to 10 seconds and particularly 2 to 8 seconds, of being placed in the oral cavity. It therefore encompasses all the types of dosage form described in the preceding paragraphs as well as any other equivalent dosage form. However, it is particularly preferred that the fast-dispersing dosage form is of the type described in U.K. Patent No. 1548022, that is, a solid fast-dispersing dosage form comprising a network of the active ingredient and a water-soluble or water-dispersible carrier which is inert towards the active ingredient, the network having been obtained by subliming solvent from a composition in the solid state, that composition comprising the active ingredient and a solution of the carrier in a solvent.

In the case of the preferred type of fast-dispersing dosage form described above, the composition will preferably contain, in addition to the active ingredient, matrix forming agents and secondary components. Matrix forming agents suitable for use in the present invention include materials derived from animal or vegetable proteins, such as the gelatins, dextrins and soy, wheat and psyllium seed proteins; gums such as acacia, guar, agar, and xanthan; polysaccharides; alginates; carboxymethylcelluloses; carrageenans; dextrans; pectins; synthetic polymers such as polyvinylpyrrolidone; and polypeptide/protein or polysaccharide complexes such as gelatin-acacia complexes.

Other matrix forming agents suitable for use in the present invention include sugars such as mannitol, dextrose, lactose, galactose and trehalose; cyclic sugars such as cyclodextrin; inorganic salts such as sodium phosphate, sodium chloride and aluminium silicates; and amino acids having from 2 to 12 carbon atoms such as a glycine, L-alanine, L-aspartic acid, L-glutamic acid, L-hydroxyproline, L-isoleucine, L-leucine and L-phenylalanine.

One or more matrix forming agents may be incorporated into the solution or suspension prior to solidification. The matrix forming agent may be present in addition to a surfactant or to the exclusion of a surfactant. In addition to forming the matrix, the matrix forming agent may aid in maintaining the dispersion of any active ingredient within the solution or suspension. This is especially helpful in the case of active agents that are not sufficiently soluble in water and must, therefore, be suspended rather than dissolved.

Secondary components such as preservatives, antioxidants, surfactants, viscosity enhancers, colouring agents, flavouring agents, pH modifiers, sweeteners or taste-masking agents may also be incorporated into the composition. Suitable colouring agents include red, black and yellow iron oxides and FD & C dyes such as FD & C blue No. 2 and FD & C red No. 40 available from Ellis & Everard. Suitable flavouring agents include mint, raspberry, liquorice, orange, lemon, grapefruit, caramel, vanilla, cherry and grape flavours and combinations of these. Suitable pH modifiers include citric acid, tartaric acid, phosphoric acid, hydrochloric acid and maleic acid. Suitable sweeteners include aspartame, acesulfame K and thaumatic. Suitable taste-masking agents include sodium bicarbonate, ion-exchange resins, cyclodextrin inclusion compounds, adsorbates or microencapsulated actives.

The present invention might for example, be used for the delivery of vaccines designed to prevent or reduce the symptoms of diseases of which the following is a representative but not exclusive list:
Influenza, Tuberculosis, Meningitis, Hepatitis, Whooping Cough, Polio, Tetanus, Diphtheria, Malaria, Cholera, Herpes, Typhoid, HIV, AIDS, Measles, Lyme disease, Travellers' Diarrhoea, Hepatitis A, B and C, Olitis Media, Dengue Fever, Rabies, Parainfluenza, Rubella, Yellow Fever, Dysentery, Legionnaires Disease, Toxoplasmosis, Q-Fever, Haemorrhegic Fever, Argentina Haemorrhagic Fever, Caries, Chagas Disease, Urinary Tract Infection caused by E.coli, Pneumoccoccal Disease, Mumps, and Chikungunya.

It can be used to prevent or reduce the symptoms of other disease syndromes of which the following is a representative but not exclusive list of causative organisms:
Vibrio species, Salmonella species, Bordetella species, Haemophilus species, Toxoplasmosis gondii, Cytomegalovirus, Chlamydia species, Streptococcal species, Norwalk Virus, Escherischia coli, Helicobacter pylori, Rotavirus, Neisseria gonorrhae, Neisseria meningiditis, Adenovirus, Epstein Barr Virus, Japanese Encephalitis Virus, Pneumocystis carini, Herpes simplex, Clostridia species, Respiratory Syncytial Virus, Klebsielia species, Shigella species, Pseudomonas aeruginosa, Parvovirus, Campylobacter species, Rickettsia species, Varicella zoster, Yersinia species, Ross River Virus, J.C. Virus, Rhodococcus equi, Moraxella catarrhalis, Borrelia burgdorferi and Pasteurella haemolytica.

The invention can also be used with vaccines directed to non-infectious immuno-modulated disease conditions such as topical and systematic allergic conditions such as Hayfever, Asthma, Rheumatoid Arthritis and Carcinomas.

Prospective vaccines for veterinary use include those directed to Coccidiosis, Newcastle Disease, Enzootic pneumonia, Feline leukaemia, Atrophic rhinitis, Erysipelas, Foot and Mouth disease, Swine, pneumonia, and other disease conditions and other infections and auto-immune disease conditions affecting companion and farm animals.

In a preliminary test the immunogenicity of tetanus toxoid (TT) in twenty-five rabbits was studied following oral delivery in fast dispersing dosage forms (FDDF) of the kind described in British Patent No. 1,548,022. As references, similar tests were conducted using oral administration of TT in solution and intramuscular administration by injection of TT adsorbed to aluminium hydroxide. The administered formulations are set out in Table 1 in which the TT concentration is expressed as the concentration of TT protein. The adjuvants used in formulations 1 to 3, PLSP and Chitosan, are discussed in more detail in published Patent Specification Nos. W097/02810 and WO90/09780. A summary of the dose groups is given in Table 2. Oral administration of formulations Nos 1 to 3 was by placement of the FDDF unit at the rear of the tongue after spraying the oral cavity with 0.12ml. of UHP water after which the oral cavity was again sprayed with 0.06ml. of UHP water. Formulation 4 was delivered in a dose of 0.5ml by syringe delivered to the rear of the tongue. Formulation 5 was delivered by injection of a 0.2ml dose to the quadriceps (front thigh) to muscles of the left hind limb. Prior to each dose administration, and at termination blood and saliva samples were collected. The dosing and sampling schedule is set out in Table 3.

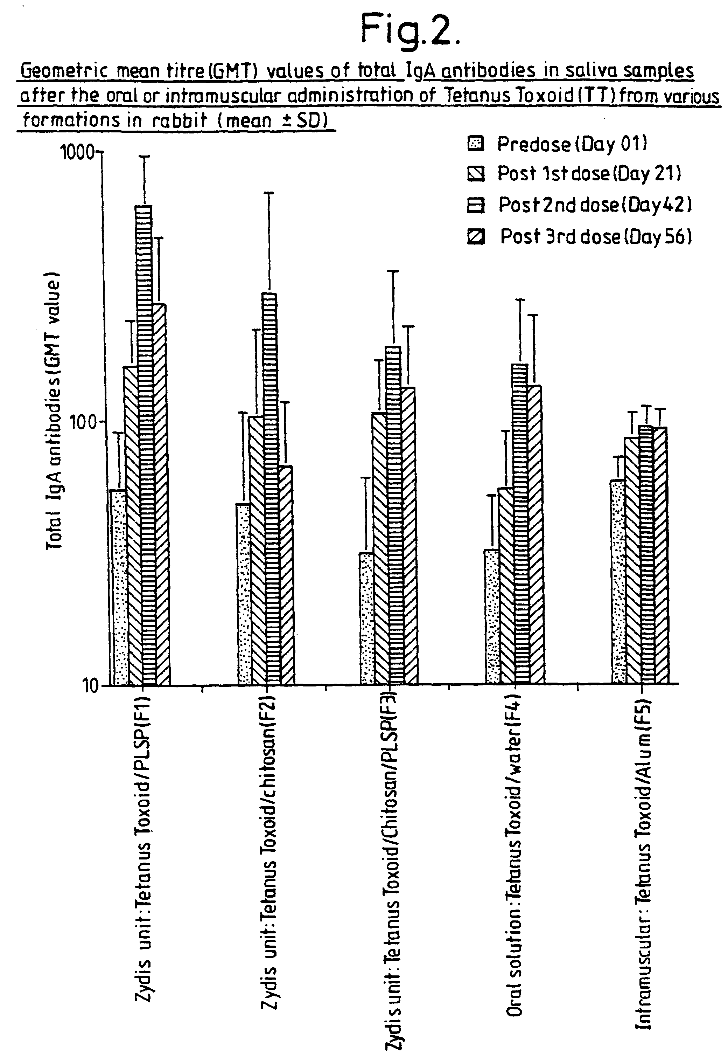

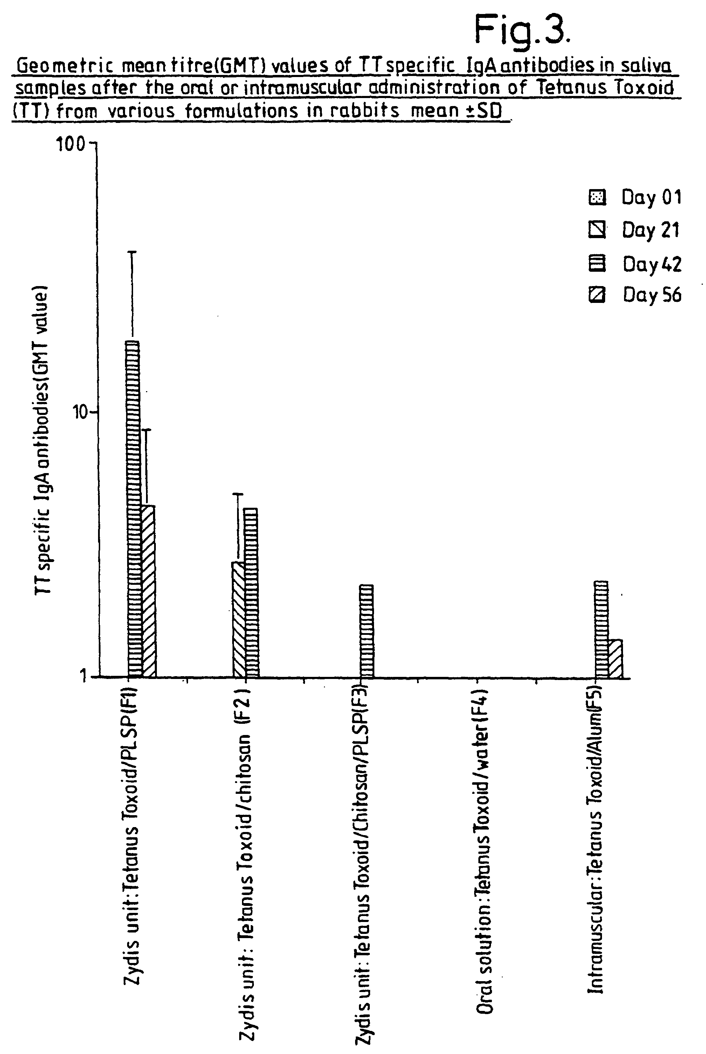

Figure 2 is a block diagram showing the geometric mean titre values of total IgA antibodies in saliva samples after administration of TT in the various formulations of Table 1 above (mean ± SD). As can be seen from the diagram (the ordinate is on a logarithmic scale) the peak IgA values achieved using formulations 1 to 3 are significantly better than those for formulation 5 with formulation 1 providing the best figures by a considerable margin. Similar tests were conducted to monitor TT specific antibodies. The results are illustrated in Figure 3. Even on a lower logarithmic scale formulations 1 and 2 show significant improvement in immune response relative to the intramuscular delivered dosage, formulations 5.

It should be noted that only saliva samples that exhibited a positive response to the assay text were recorded. This explains the apparent absence of any immune response at some stages for some formulations, and the apparent absence of response at any stage for formulation 4. The tests showed some response at these stages, but not any that were felt to the statistically significant. We were satisfied on the basis of these tests that the improved immune response exhibited by formulations 1 and 2 in Figure 3 demonstrates the potential benefit of administering vaccines with one or more adjuvants in a fast dispensing dosage form, by oral delivery.

**Table 1**

| **Formulation** **No.** | **Type of** **formulation** | **Outline Composition** | **Route of** **administration** |
|---|---|---|---|
| 1 | FDDF unit | 0.4 mg TT/PLSP/Gelatin/Mannitol | Oral |
| 2 | FDDF unit | 0.4 mg TT/Chitosan/Gelatin/Mannitol | Oral |
| 3 | FDDF unit | 0.4 mg TT/Chitosan/PLSP/Gelatin/Mannitol | Oral |
| 4 | Solution | 0.8 mg/ml TT in water | Oral |
| 5 | Suspension | 0.4 mg/ml TT/alum | IM |

**Table 2**

| **Formulation** **or Group No.** | **Type of** **formulation** **[Route]** | **Rabbit** **No.** | **TT** **(mg/rabbit)** | **Chitosan** **(mg/rabbit)** | **PLSP** **(mg/rabbit)** | **Gelatin** **(mg/rabbit)** | **Mannitol** **(mg/rabbit)** | **Alum** **(mg/rabbit)** |
|---|---|---|---|---|---|---|---|---|
| 1 | FDDF unit [oral] | 1-5 | 0.4 | - | 10 | 15 | 15 | - |
| 2 | FDDF unit [oral] | 6-10 | 0.4 | 5 | - | 5 | 5 | - |
| 3 | FDDF unit [oral] | 11-15 | 0.4 | 5 | 10 | 5 | 5 | - |
| 4 | Solution [oral] | 16-20 | 0.4 | - | - | - | - | - |
| 5 | Suspension [IM] | 21-25 | 0.08 | - | - | - | - | 4.8 |

**Table 3**

| **Study Date** | **Study Day** | **Procedure** |
|---|---|---|
| 31/07/98 | 1 | Collect pre-dose saliva samples from rabbits 1-25 Dose rabbits 1-25 with the appropriate formulations (refer to Table 2) |
| 20/08/98 | 21 | Collect pre-dose saliva samples from rabbits 1-25 Dose rabbits 1-25 with the appropriate formulation (refer to Table 2) |
| 10/09/98 | 42 | Collect pre-dose saliva samples from rabbits 1-25 Dose rabbits 1-25 with the appropriate formulation (refer to Table 2) |
| 24/09/98 | 56 | Collect terminal saliva samples from rabbits 1-25 |

## Claims

1. A vaccine composition comprising an immunogenic amount of an antigenic preparation in an orally administered solid dispersing form designed to disintegrate rapidly in the oral cavity, the composition including an adjuvant to enhance absorption of the antigen at the mucosal tissues of the buccopharyngeal, wherein said adjuvant comprises biodegradable polymeric material particles in which the antigenic preparation is encapsulated or on which the antigenic preparation is absorbed, **characterised in that** in use said solid dispersing form disintegrates rapidly in the oral cavity whereby to coat and be retained in contact with the mucosal tissue of the buccopharyngeal region prior to absorption by said mucosal tissue.

2. A vaccine composition according to claim 1, wherein the polymeric particles are formed of a poly(lactic acid) or a poly(lactide-co-glycide), or a combination thereof.

3. A vaccine composition according to claim 1, wherein the polymeric particle is latex.

4. A vaccine composition according to any preceding claim, including a mucoadhesive substance.

5. A vaccine composition according to any preceding claim, in which the solid dispersing form is a low density matrix tablet.

6. A vaccine composition according to claim 5, wherein the low density matrix is formed by removal of solvent by lyophilisation from a frozen suspension.

7. A vaccine composition according to claim 5, wherein the low density matrix is formed by removal of solvent from a frozen suspension by contact with a second solvent, in which the matrix forming materials are insoluble.

8. A vaccine composition according to claim 5, wherein the low density matrix is formed by compacting finely divided extruded materials.

9. A vaccine composition according to claim 5, wherein the low density matrix is formed by loosely compacting particles formed by spray-coating, spray-drying, lyophilisation, spray-chilling, coacervation, or fluid-bed drying.

10. A vaccine composition according to claim 5, wherein the low density matrix is formed by gelling a suspension and then removing solvent by drying.

11. A vaccine composition according to any preceding claim, wherein the solid dispersing form disintegrates within one minute in the oral cavity.

## Patentansprüche

1. Impfstoffzusammensetzung einer immunogenen Menge eines antigenen Präparats, in einer oral verabreichbaren festen dispergierten Form so konzipiert, dass es in der Mundhöhle rasch zerfällt, welche Zusammensetzung ein Adjuvans einschließt, um die Aufnahme des Antigens auf der Schleimhaut des Mund-Rachenraums zu verstärken, wobei das Adjuvans Partikel aus biozersetzbarem polymeren Material aufweist, in denen das antigene Präparat gekapselt oder auf denen das antigene Präparat absorbiert ist; **dadurch gekennzeichnet, dass** die feste dispergierte Form bei Gebrauch in der Mundhöhle rasch zerfällt, wodurch sie die Schleimhaut des Mund-Rachenbereichs überzieht und vor der Aufnahme durch die Schleimhaut mit ihr in Kontakt gehalten wird.

2. Impfstoffzusammensetzung nach Anspruch 1, worin die polymeren Partikel aus einer Polymilchsäure oder einem Poly(lactid-co-glycid) oder einer Kombination davon gebildet sind.

3. Impfstoffzusammensetzung nach Anspruch 1, worin das polymere Partikel Latex ist.

4. Impfstoffzusammensetzung nach einem der vorgenannten Ansprüche, einschließend eine mukoadhäsive Substanz.

5. Impfstoffzusammensetzung nach einem der vorgenannten Ansprüche, worin die feste dispergierte Form eine Tablette mit einer Matrix geringer Dichte ist.

6. Impfstoffzusammensetzung nach Anspruch 5, worin die Matrix geringer Dichte erzeugt wird, indem Lösemittel durch Lyophilisierung aus einer tiefgefrorenen Suspension entfernt wird.

7. Impfstoffzusammensetzung nach Anspruch 5, worin die Matrix geringer Dichte erzeugt wird, indem Lösemittel aus einer tiefgefrorenen Suspension durch Kontakt mit einem zweiten Lösemittel entfernt wird, worin die Materialien, die die Matrix erzeugen, unlöslich sind.

8. Impfstoffzusammensetzung nach Anspruch 5, worin die Matrix geringer Dichte erzeugt wird durch Kompaktieren von fein verteilten extrudierten Materialien.

9. Impfstoffzusammensetzung nach Anspruch 5, worin die Matrix geringer Dichte erzeugt wird durch loses Kompaktieren von Partikeln, die durch Sprühbeschichten, Sprühtrocknen, Lyophilisierung, Sprühgefrieren, Koazervierung oder durch Wirbelschichttrocknen erzeugt werden.

10. Impfstoffzusammensetzung nach Anspruch 5, worin die Matrix geringer Dichte erzeugt wird durch Gelbildung einer Suspension und anschließendes Entfernen des Lösemittels durch Trocknen.

11. Impfstoffzusammensetzung nach einem der vorgenannten Ansprüche, worin die feste dispergierte Form innerhalb einer Minute in der Mundhöhle zerfällt.

## Revendications

1. Composition vaccinale comprenant une quantité immunogène d'une préparation antigénique sous une forme dispersante solide administrée oralement, conçue pour se désintégrer rapidement dans la cavité orale, la composition incluant un adjuvant pour améliorer l'absorption de l'antigène aux tissus muqueux du bucco-pharynx, en quoi ledit adjuvant est composé de particules de matière polymérique biodégradable dans lesquelles la préparation antigénique est encapsulée ou sur lesquelles la préparation antigénique est absorbée, **caractérisée en ce que** lorsqu'elle est utilisée, ladite forme dispersante solide se désintègre rapidement dans la cavité orale et, se faisant, enduit et est retenue au contact du tissu muqueux de la région bucco-pharyngée avant absorption par ledit tissu muqueux.

2. Composition vaccinale selon la revendication 1, en quoi les particules polymériques sont formées d'un poly(acide lactique) ou d'un poly(lactide-co-glycide), ou d'une combinaison de cela.

3. Composition vaccinale selon la revendication 1, en quoi la particule polymérique est du latex.

4. Composition vaccinale selon n'importe laquelle des revendications précédentes, incluant une substance muco-adhésive.

5. Composition vaccinale selon n'importe laquelle des revendications précédentes, dans laquelle la forme dispersante solide est un comprimé à matrice de faible densité.

6. Composition vaccinale selon la revendication 5, en quoi la matrice de faible densité est formée en enlevant le solvant par lyophilisation à partir d'une suspension congelée.

7. Composition vaccinale selon la revendication 5, en quoi la matrice de faible densité est formée en enlevant le solvant d'une suspension congelée par contact avec un second solvant, dans lequel les matières formant la matrice sont insolubles.

8. Composition vaccinale selon la revendication 5, en quoi la matrice de faible densité est formée en compactant des matières extrudées divisées finement.

9. Composition vaccinale selon la revendication 5, en quoi la matrice de faible densité est formée en compactant de manière lâche des particules formées par revêtement par pulvérisation, séchage par pulvérisation, lyophilisation, glaçage par pulvérisation, coacervation ou séchage en lit fluidifié.

10. Composition vaccinale selon la revendication 5, en quoi la matrice de faible densité est formée en gélifiant une suspension et ensuite en enlevant le solvant par séchage.

11. Composition vaccinale selon n'importe laquelle des revendications précédentes, en quoi la forme dispersante solide se désintègre en une minute dans la cavité orale.
